# FASCICULE DE BREVET EUROPEEN

(11) **EP 4 072 636 B1**
(45) Date de publication et mention de la délivrance du brevet: **07.08.2024**
(21) Numéro de dépôt: 20821219.1
(22) Date de dépôt: 11.12.2020
(51) Int. Cl.: A61M 15/00, A61M 16/04

(54) **EMBOUT BUCCAL POUR DISPOSITIF DE DISTRIBUTION DE PRODUIT**
MUNDSTÜCK FÜR EINE PRODUKTAUSGABEVORRICHTUNG
MOUTHPIECE FOR A PRODUCT DISPENSING DEVICE

(30) Priorité: 11.12.2019 FR 1914200
(43) Date de publication de la demande: 19.10.2022
(73) Titulaire: NEMERA LA VERPILLIERE, 38290 La Verpillière (FR); Université de Tours, 37020 Tours Cedex 1 (FR); Institut National de la Santé et de la Recherche Médicale (INSERM), 75013 Paris (FR)
(72) Inventeur: REGARD, Alain, 01700 BEYNOST (FR); GREVIN, Guillaume, 38080 L ISLE D ABEAU (FR); DELVALAC, Sébastien, 69007 LYON (FR); LE PENNEC, Déborah, 37260 MONTS (FR); VECELLIO, Laurent, 37170 CHAMBRAY LES TOURS (FR)
(74) Mandataire: LLR
(86) Numéro de dépôt international: PCT/EP2020/085851
(87) Numéro de publication internationale: WO 2021/116458

(56) Documents cités:
- WO-A2-2004/103447
- US-A- 5 507 278
- US-A- 5 746 197
- US-B2- 7 676 276
- US-B2- 9 302 060

## Description

L'invention concerne un embout buccal pour dispositif de distribution de produit.

On connaît déjà dans l'état la technique, par exemple des documents US 5 746 197 et US 5 507 278, des embouts buccaux pour dispositifs de distribution de produit. Par exemple, on connaît de tels embouts pour des dispositifs d'administration d'aérosol, ces dispositifs étant généralement constitués d'un générateur de particules, par exemple dont la taille est comprise entre 10 nanomètres et 200 micromètres, d'une source de pression pour transporter les particules et d'un embout buccal, lequel permet l'administration de l'aérosol par la bouche. La source de pression peut être endogène, constituée par exemple par une pression d'aspiration obtenue par une inspiration de l'utilisateur par la bouche. La source de pression peut alternativement être exogène, constituée par exemple d'un réservoir de gaz sous pression.

Les particules, par exemple d'un médicament, peuvent être sous forme liquide ou sous forme solide. Dans le cas de particules à l'état liquide, le générateur de particules se présente par exemple sous la forme d'un atomiseur permettant la formation d'un spray. Dans le cas de particules à l'état solide, le générateur de particules délivre par exemple une quantité prédéterminée de poudre sur un support, lequel est ensuite par exemple soumis à l'action d'un flux propulseur.

Le médicament peut dans certains cas être déposé dans les cavités nasales après passage par la bouche. Alternativement, le médicament peut être déposé dans les poumons en passant par la bouche.

On connaît ainsi un dispositif d'administration d'aérosol tel que divulgué dans le document FR2954707. Un tel dispositif comporte un embout buccal permettant l'administration d'un aérosol par la bouche durant une phase d'expiration nasale ou durant une phase de pause respiratoire précédant l'expiration nasale. Dans ce dispositif, l'embout buccal est étanche à l'air et pénètre au-delà des dents du patient selon une certaine longueur. Ainsi, dans ce cas, l'embout buccal constitue un moyen d'administration de l'aérosol à visée nasale, rhinopharyngée ou nasosinusienne. L'aérosol contenant les particules est ainsi acheminé successivement dans la bouche, le rhinopharynx puis les fosses nasales et les sinus, jusqu'à son échappement par au moins une narine du patient.

Toutefois, le dépôt de produit dans la bouche peut ne pas être souhaitable. En effet, un tel dépôt réduit l'efficacité de la distribution du produit, donc la dose effectivement reçue par l'organe cible, du fait du dépôt de produit dans la bouche au lieu de la zone de traitement souhaitée. En outre, le produit peut également altérer ou à minima gêner le sens du goût d'un utilisateur.

Ainsi, compte-tenu de leur forme, les embouts buccaux actuels n'évitent pas ou que très partiellement le dépôt de produit dans la bouche.

L'invention a notamment pour but de proposer un embout buccal, lequel garantit qu'un maximum de la dose distribuée est reçue par l'organe cible.

A cet effet, l'invention a pour objet un embout buccal pour un dispositif de distribution de produit, comportant :
- un conduit de distribution de produit définissant un axe longitudinal et comprenant une ouverture de raccordement configurée pour être raccordée à un organe de distribution du dispositif de distribution de produit, et une ouverture de distribution configurée pour déboucher librement dans la bouche d'un utilisateur, et
- un logement lingual configuré pour recevoir au moins une extrémité de la langue de l'utilisateur, comportant une ouverture linguale autorisant l'insertion de la langue dans le logement lingual et une surface de contact configurée pour former une cible pour une extrémité de la langue de l'utilisateur.

Ainsi, on propose de réaliser l'invention en ménageant un logement pour la langue de l'utilisateur, lequel comporte en outre une cible haptique pour une extrémité de la langue de l'utilisateur, afin que la langue de l'utilisateur soit incitée à être avancée, donc abaissée, de façon que les voies respiratoires ou aériennes se trouvant derrière la langue soient davantage dégagées. Ceci permet de minimiser le dépôt de produit dans la bouche, par exemple sur la langue.

L'utilisateur, en atteignant la cible avec sa langue, ou a minima en tentant de l'atteindre avec sa langue, s'assure ainsi qu'un maximum de la dose distribuée atteint l'organe cible et qu'il y a moins de déperdition dans la bouche.

Suivant d'autres caractéristiques optionnelles de l'embout buccal, prises seules ou en combinaison :
- le logement lingual est au moins partiellement adjacent au conduit de distribution. Ainsi, l'encombrement de l'embout buccal dans la bouche est diminué.
- l'embout buccal comporte en outre un ouvre-bouche entourant au moins en partie le conduit de distribution et le logement lingual. Ainsi, du fait de l'ouvre-bouche, l'utilisateur est contraint à ouvrir en grand la bouche, de sorte que la mâchoire inférieure de l'utilisateur est abaissée, ce qui augmente l'accessibilité du fond de la bouche et améliore par conséquent la distribution de produit dans des zones situées au-delà de la bouche, en d'autres termes en aval de la bouche dans le sens de l'écoulement de distribution de produit. En outre, l'ouvre-bouche permet de positionner l'embout buccal. On comprend que l'ouvre-bouche sert avantageusement de support pour le conduit de distribution et le logement lingual.

- l'ouvre-bouche comprend une paroi périphérique de préférence conformée sous forme de surface de révolution ou de cylindre à section transversale elliptique ou encore ovoïde, de préférence sous forme de cylindre droit à section transversale circulaire. Ainsi, lorsque l'ouvre-bouche est en forme de O, une étanchéité peut en outre être obtenue entre les lèvres de l'utilisateur et l'ouvre-bouche, évitant ainsi que du produit sorte de la cavité buccale. Une surface de révolution est obtenue par rotation d'une courbe ou d'une droite autour d'un axe de révolution. Une surface de révolution est par exemple un cylindre, lequel peut par exemple être un cylindre droit à section circulaire. Dans ce cas, l'appui est plus confortable. Alternativement, la surface de révolution peut par exemple être conique, et permet ainsi à l'embout buccal de s'adapter à différentes tailles de bouches.
- l'embout buccal comprend en outre une surface d'appui frontale pour les lèvres de l'utilisateur. Ainsi, l'embout buccal est positionné précisément axialement dans la bouche de l'utilisateur, et ce de manière simple et répétable pour l'utilisateur. L'étanchéité de la distribution est par ailleurs favorisée.
- la surface d'appui frontale est comprise dans un plan défini par la surface de contact. Ainsi, le nettoyage du dispositif est facilité, tout particulièrement le nettoyage du logement lingual entre deux utilisations.
- l'embout buccal comprend une plaque comportant la surface d'appui frontale et comportant une unique ouverture délimitant l'ouverture de raccordement. Ainsi, du fait de l'ouverture unique, l'air ambiant n'exerce pas d'influence pouvant affecter négativement la distribution de produit et par conséquent le dépôt de produit dans la bouche.
- un angle formé entre l'axe longitudinal du conduit de distribution et la surface d'appui frontale possède une valeur comprise entre 90 degrés et 120 degrés, de préférence entre 92 degrés et 100 degrés, plus préférentiellement voisine de 97,5 degrés. Ainsi, l'angle formé par le conduit de distribution par rapport à la bouche de l'utilisateur permet d'orienter le produit vers le fond de la gorge et donc de diminuer le dépôt de produit dans le haut de la bouche de l'utilisateur. On notera que l'axe longitudinal du conduit de distribution peut être un axe de révolution du conduit de distribution lorsque celui, ou plus généralement s'il n'est pas de révolution, un axe central du conduit de distribution.
- la surface d'appui frontale est sensiblement perpendiculaire à un axe central de l'ouvre-bouche. Ainsi, l'embout buccal est positionné plus précisément axialement dans la bouche de l'utilisateur.
- le logement lingual est au moins partiellement aligné avec une partie inférieure du conduit de distribution. Ainsi, la partie inférieure du conduit de distribution permet de guider la langue de l'utilisateur de manière confortable en direction du logement lingual.
- le conduit de distribution comporte en outre au moins un orifice de passage d'air, lequel est disposé préférentiellement sur la partie supérieure du conduit de distribution opposée au logement lingual. Ainsi, la distribution de produit est davantage accélérée et améliorée du fait de l'effet Venturi, causé à l'écoulement de la distribution de produit par aspiration d'air à travers l'orifice de passage d'air.
- le conduit de distribution présente une longueur, entre la surface d'appui frontale et l'ouverture de distribution, comprise entre 30 millimètres et 70 millimètres, de préférence entre 50 millimètres et 60 millimètres, plus préférentiellement voisine de 55 millimètres. Ainsi, la longueur du conduit est optimale pour limiter le dépôt de produit dans la bouche, tout en restant confortable pour l'utilisateur.
- la paroi interne du conduit de distribution est conformée sous forme de surface de révolution ou de cylindre à section transversale elliptique, de préférence sous forme de cylindre droit à section transversale elliptique. Ainsi, l'écoulement de distribution de produit est amélioré. Une surface de révolution est obtenue par rotation d'une courbe ou d'une droite autour d'un axe de révolution. Une surface de révolution est par exemple un cylindre, lequel peut par exemple être un cylindre droit à section circulaire. Alternativement, la surface de révolution peut par exemple être conique.
- l'ouverture de raccordement est configurée pour recevoir un organe de distribution du dispositif de distribution de produit ou le dispositif de distribution de produit luimême. Ainsi, le conduit de distribution est raccordé de manière particulièrement simple à l'organe de distribution du dispositif de distribution de produit. En d'autres termes, l'ouverture de raccordement se présente sous la forme d'un logement de réception, lequel est situé en amont de l'ouverture de distribution de produit, en considérant le sens de l'écoulement de distribution du produit.
- l'embout buccal comporte des moyens de fixation amovibles à un organe de distribution du dispositif de distribution de produit, de préférence comprenant l'ouverture de raccordement, laquelle présente préférentiellement une forme conique. Ainsi, l'embout buccal peut facilement être monté sur un dispositif de distribution de produit existant, par exemple déjà doté d'un embout buccal. Du fait des moyens de fixation amovibles, l'embout peut également être démonté, par exemple pour permettre son nettoyage. Lorsque l'ouverture de raccordement est de forme conique, la fixation amovible de l'organe de distribution et de l'embout buccal est réalisée de manière simple et étanche par serrage de l'organe de distribution dans l'ouverture de raccordement.
- le logement lingual comprend des moyens de détection de la position de la langue, de préférence un capteur de proximité ou un capteur de contact sur la surface de contact. Ainsi, il est possible de transmettre une information relative au positionnement de la langue de l'utilisateur à l'utilisateur ou au dispositif de distribution de produit, par exemple l'utilisateur ou le dispositif de distribution de produit pouvant alors déclencher la distribution de produit, en cas de détection d'un positionnement correct de la langue de l'utilisateur.
- le logement lingual comprend des moyens d'indication d'un positionnement correct de la langue de l'utilisateur, de préférence les moyens d'indication comprenant une surface texturée agencée sur la surface de contact. Ainsi, l'information relative au positionnement correct de la langue de l'utilisateur est réalisée de manière particulièrement simple. Par surface texturée, il faut notamment comprendre une surface dont la rugosité ou la présence de reliefs est suffisante pour permettre une détection tactile par la langue de l'utilisateur.

L'invention a également pour objet un dispositif de distribution de produit, lequel comporte :
- un organe de distribution, et
- un embout buccal du type susmentionné.

L'invention a encore pour objet un procédé de distribution d'un produit au moyen d'un dispositif de distribution de produit du type susmentionné, comprenant au moins les étapes suivantes :
- insertion de l'embout buccal dans la bouche d'un utilisateur,
- insertion de la langue de l'utilisateur dans le logement lingual, et
- déclenchement de la distribution de produit lorsque la langue de l'utilisateur est en contact avec la surface de contact.

### Brève description des figures

L'invention sera mieux comprise à la lecture de la description qui va suivre donnée uniquement à titre d'exemple et faite en se référant aux dessins annexés dans lesquels :
[Fig. 1] la figure 1 est une vue en perspective arrière d'un embout buccal selon un premier mode de réalisation ;
[Fig. 2] la figure 2 est une vue en perspective avant de l'embout buccal selon le premier mode de réalisation illustré sur la figure 1 ;
[Fig. 3] la figure 3 est une vue en coupe de l'embout buccal selon le premier mode de réalisation illustré sur la figure 1 ;
[Fig. 4] la figure 4 est une vue en perspective arrière d'un dispositif de distribution de produit comprenant un embout buccal selon le premier mode de réalisation ;
[Fig. 5] la figure 5 est une vue en coupe d'un dispositif de distribution de produit comprenant un embout buccal selon le premier mode de réalisation et un organe de distribution selon une première variante ;
[Fig. 6] la figure 6 est une vue en coupe d'un deuxième dispositif de distribution de produit comprenant un embout buccal selon le premier mode de réalisation et un organe de distribution selon une seconde variante ;
[Fig. 7] la figure 7 est une vue en perspective arrière d'un embout buccal selon un deuxième mode de réalisation ;
[Fig. 8] la figure 8 est une vue en perspective arrière d'un embout buccal selon un troisième mode de réalisation ;
[Fig. 9] la figure 9 est une vue en section du deuxième dispositif de distribution de produit illustré sur la figure 6, dans lequel l'embout buccal est inséré dans la bouche d'un utilisateur.

### Description détaillée

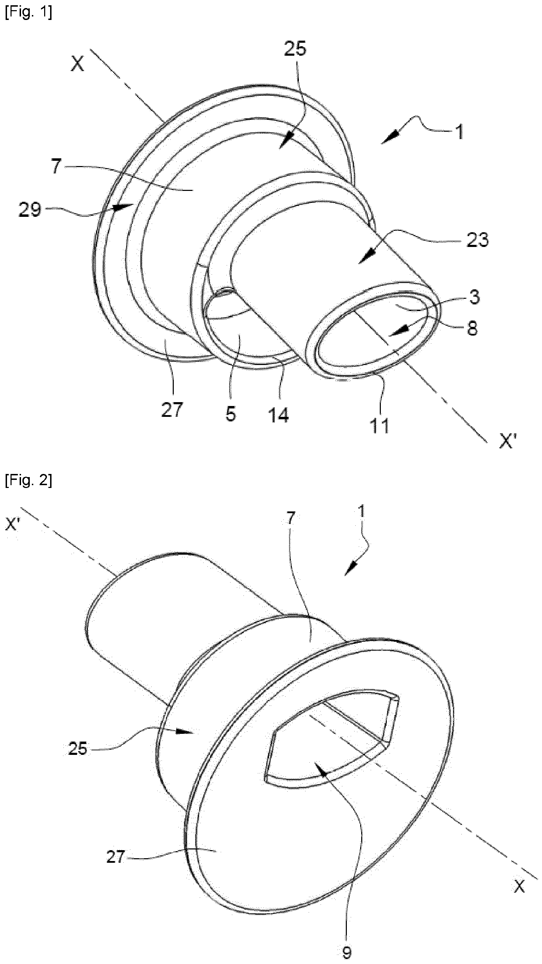

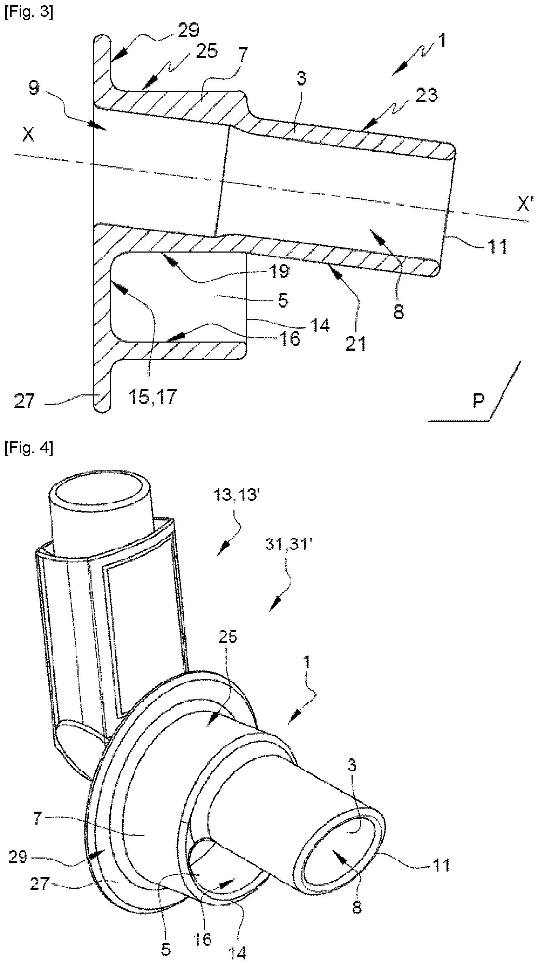

On a représenté sur les figures 1 à 3 un embout buccal selon un premier mode de réalisation de l'invention, désigné par la référence générale 1. L'embout buccal 1 est sensiblement symétrique par rapport à un plan de symétrie P, comme illustré sur la figure 3.

Cet embout buccal 1 comporte un conduit de distribution 3 et un logement lingual 5. Il comporte en outre un ouvre-bouche 7.

Le conduit de distribution 3 a pour fonction de distribuer un produit, comme par exemple un aérosol. Le conduit de distribution 3 s'étend longitudinalement, et définit ainsi un axe longitudinal XX'. Dans ce premier mode de réalisation, le conduit de distribution 3 se présente sous forme de cylindre creux à section elliptique. Ainsi, la paroi interne 8 du conduit de distribution 3 est conformée sous forme de cylindre droit à section transversale elliptique.

Le conduit de distribution 3 comprend une ouverture de raccordement 9 et une ouverture de distribution 11.

L'ouverture de raccordement 9 est configurée pour être raccordée à un organe de distribution 13, 13', comme illustré sur les figures 4 à 6. L'organe de distribution 13, 13' a pour fonction de distribuer un produit, par exemple lors d'un actionnement par un utilisateur.

L'ouverture de distribution 11 est configurée pour déboucher librement dans la bouche d'un utilisateur. Ainsi, l'ouverture de distribution 11 constitue l'extrémité aval du conduit de distribution 3, dans le sens de l'écoulement du produit lors d'une distribution de produit.

Le logement lingual 5 est configuré pour recevoir au moins une extrémité de la langue de l'utilisateur. Le logement lingual 5 comporte une ouverture linguale 14, laquelle autorise l'insertion de la langue de l'utilisateur dans le logement lingual 5. Le logement lingual 5 comporte également une surface de contact 15, laquelle est configurée pour former une cible pour une extrémité de la langue de l'utilisateur. Dans ce premier mode de réalisation, la surface de contact 15 se trouve au fond du logement lingual 5. De manière alternative ou complémentaire, la surface de contact peut être agencée sur la surface latérale 16 du logement lingual 5. Par exemple, le logement lingual 5 présente une profondeur de 20 millimètres.

Le logement lingual 5 comporte en outre des moyens d'indication d'un positionnement correct de la langue de l'utilisateur. Dans l'exemple illustré sur les figures 1 à 3, les moyens d'indication comprennent une surface texturée 17 agencée sur la surface de contact 15. De manière alternative ou complémentaire, le logement lingual 5 peut comprendre des moyens de détection de la position de la langue, comme par exemple un capteur de proximité ou un capteur de contact sur la surface de contact 15.

Le logement lingual 5 est au moins partiellement adjacent au conduit de distribution 3. Le logement lingual 5 est en outre au moins partiellement aligné avec une partie inférieure du conduit de distribution 3. Plus précisément, comme illustré sur la figure 3, la partie supérieure 19 de la surface latérale 16 du logement lingual 5 est raccordée à la partie inférieure 21 de la surface périphérique 23 du conduit de distribution 3.

Comme illustré notamment sur les figures 1 à 3, l'ouvre-bouche 7 comprend une paroi périphérique 25 sous forme de cylindre droit à section transversale circulaire. Par exemple, le diamètre de la paroi périphérique 25 est égal à 40 millimètres.

L'ouvre-bouche 7, comme cela est visible notamment sur la figure 3, entoure au moins en partie le conduit de distribution 3 et le logement lingual 5. Plus précisément, le logement lingual 5 est disposé dans l'ouvre-bouche 7, et le conduit de distribution 3 fait partiellement saillie depuis l'ouvre-bouche 7, du côté de l'ouverture linguale 14 du logement lingual 5. Par exemple, les dimensions extérieures de la section transversale elliptique de la partie périphérique 23 du conduit de distribution 3 qui fait saillie depuis l'ouvre-bouche 7 sont les suivantes : grand axe égal à 29 millimètres, petit axe égal à 20 millimètres. Par exemple, la longueur de la partie périphérique 23 du conduit de distribution 3 qui fait saillie depuis l'ouvre-bouche 7 est d'environ 30 millimètres, notamment égale à 28,7 millimètres dans la partie inférieure 21 de la surface périphérique 23 dans le plan de symétrie P. Par exemple, la partie périphérique 23 du conduit de distribution 3 présente une épaisseur allant de 0,8 millimètres à 2,5 millimètres, préférentiellement proche de 1,3 millimètres. Le logement lingual 5 présente un contour épousant la forme de l'ouvre-bouche 7 et du conduit de distribution 3, et présente ainsi une forme sensiblement de portion d'anneau, comme visible sur la figure 1, dont la hauteur verticale maximale est par exemple de 13,4 millimètres dans le plan de symétrie P.

L'embout buccal 1 comprend également une plaque 27, laquelle comprend une surface d'appui frontale 29 pour les lèvres de l'utilisateur.

La plaque 27 est disposée sur une extrémité de l'ouvre-bouche 7, du côté opposé à l'ouverture linguale 14 du logement lingual 5. La surface d'appui frontale 29 est ainsi perpendiculaire à un axe central de l'ouvre-bouche 7. La plaque 27 comporte en outre une unique ouverture, laquelle délimite l'ouverture de raccordement 9. Par exemple, la plaque 27 présente une épaisseur de 2,5 millimètres.

Dans l'exemple illustré, la surface d'appui frontale 29 est comprise dans un plan défini par la surface de contact 15. Ainsi, la plaque 27 porte également la surface de contact 15 se trouvant au fond du logement lingual 5.

Un angle formé entre l'axe longitudinal XX' du conduit de distribution 3 et la surface d'appui frontale 29 possède une valeur comprise entre 90 degrés et 120 degrés, de préférence entre 92 degrés et 100 degrés. Selon un exemple non illustré, l'angle possède une valeur de 92,5 degrés. Selon un autre exemple non illustré, l'angle possède une valeur de 95 degrés. Dans l'exemple illustré sur les figures, cet angle possède une valeur de 97,5 degrés.

Comme illustré sur les figures 4 à 6, l'embout buccal 1 peut constituer un élément d'un dispositif de distribution de produit 31, 31'. Ainsi, le dispositif de distribution de produit 31, 31' comporte un organe de distribution 13, 13' et un embout buccal 1. De cette manière, l'embout buccal 1 est réutilisable.

L'organe de distribution 13, 13' comporte un réservoir 33 de produit et des moyens de distribution 35, lesquels comportent par exemple une valve ou une pompe. Les moyens de distribution 35, lorsque l'organe de distribution 13, 13' est actionné, permettent de distribuer du produit en direction d'une ouverture de sortie 37 de l'organe de distribution 13, 13'. Comme illustré sur les figures 4 à 6, l'ouverture de raccordement 9 se présente sous la forme d'un logement qui possède une forme complémentaire de celle d'un organe de distribution 13,13' du dispositif de distribution de produit 31, 31'. Plus précisément, l'ouverture de raccordement 9 possède une forme complémentaire de la forme de la paroi périphérique 38 délimitant l'ouverture de sortie 37 de l'organe de distribution 13,13' du dispositif de distribution de produit 31, 31'. Ainsi, l'ouverture de raccordement 9 possède une forme complémentaire de celle de l'ouverture de sortie 37. De cette manière, l'embout buccal 1 comporte des moyens de fixation amovibles à l'organe de distribution 13, 13' du dispositif de distribution de produit 31, 31'. Par exemple, de préférence comprenant l'ouverture de raccordement 9 présente une section transversale sous forme de rectangle déformé, dont les longueurs sont formées par des arcs de cercle. En outre, l'ouverture de raccordement 9 peut présenter une forme conique.

Afin de permettre une liaison étanche par serrage de l'organe de distribution 13, 13' dans l'ouverture de raccordement 9 de l'embout buccal 1, l'ouverture de raccordement 9 présente par exemple une profondeur d'environ 20 millimètres.

Le conduit de distribution 3 présente une longueur, entre la surface d'appui frontale et l'ouverture de distribution 11, comprise entre 30 millimètres et 70 millimètres, de préférence entre 50 millimètres et 60 millimètres, dans l'exemple illustré voisine de 55 millimètres.

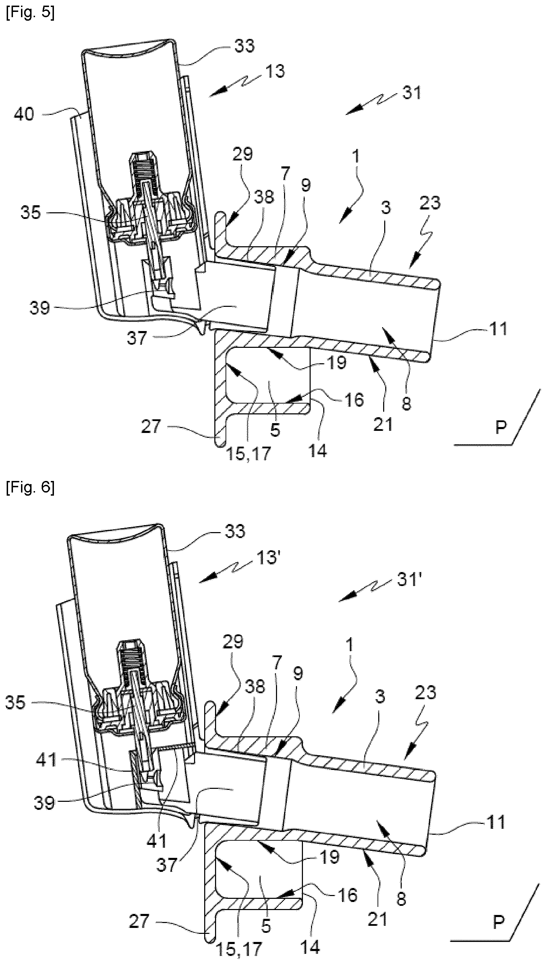

Par exemple, comme illustré sur les figures 5 et 6, la distance longitudinale entre une buse de distribution 39 des moyens de distribution 35 et l'ouverture de distribution 11 est au maximum de 90 millimètres, par exemple égale à 65 millimètres.

Dans une première variante illustrée sur la figure 5, l'organe de distribution 13 n'est pas étanche. En effet, lors du déclenchement de la distribution, de l'air extérieur, à savoir extérieur à l'utilisateur, passe depuis une ouverture d'entrée 40 de l'organe de distribution 13 vers l'ouverture de sortie 37.

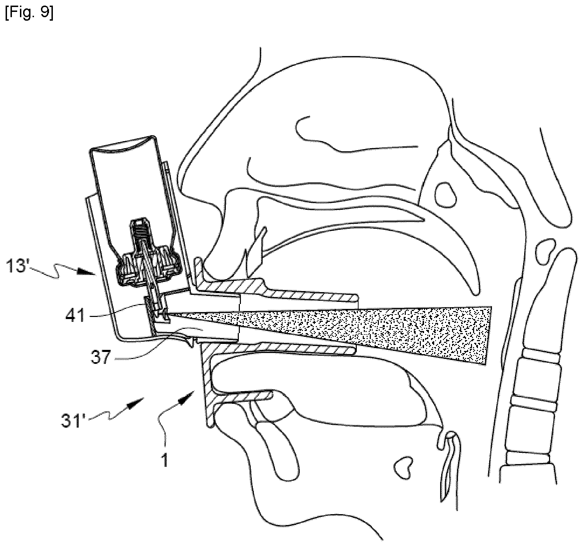

Dans une seconde variante illustrée sur la figure 6, l'organe de distribution 13' comporte une paroi interne 41, laquelle obture tout passage d'air extérieur en direction de l'ouverture de sortie 37. Ainsi, l'organe de distribution 13' permet de distribuer du produit sans que de l'air extérieur ne pénètre dans la bouche de l'utilisateur via le dispositif de distribution 31'. La figure 9 illustre un dispositif de distribution de produit 31' comportant un tel organe de distribution 13', dont l'embout buccal 1 est inséré dans la bouche d'un utilisateur.

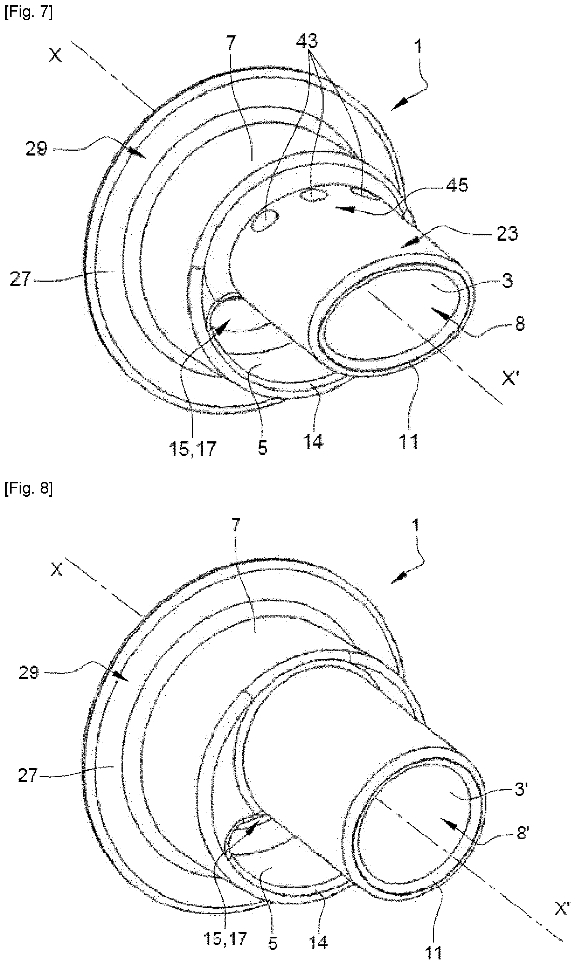

Un second mode de réalisation est illustré sur la figure 7, lequel est similaire au mode de réalisation précédemment décrit, à l'exception du fait que le conduit de distribution 3 comporte des orifices 43 de passage d'air. Dans l'exemple illustré, le conduit de distribution 3 comporte trois orifices 43 de passage d'air, lesquels sont répartis régulièrement sur la partie supérieure 45 de la surface périphérique 23 du conduit de distribution 3. De manière alternative ou complémentaire, des orifices de passage d'air pourraient être disposé sur une ou les parties latérales de la surface périphérique 23 du conduit de distribution 3. La partie supérieure 45 est opposée au logement lingual 5, en d'autres termes est opposée à la partie inférieure 21 de la surface périphérique 23 du conduit de distribution 3. Dans l'exemple illustré sur la figure 7, l'axe des orifices 43 de passage d'air appartient à un même plan, lequel est orthogonal au plan de symétrie P de l'embout buccal 1. Les orifices 43 de passage d'air sont disposés à proximité de l'ouvre-bouche 7, de manière à ce que lorsque l'utilisateur ferme les lèvres sur l'ouvre-bouche 7, l'air interne de la bouche de l'utilisateur peut passer à travers les orifices 43. Du fait de leur disposition à proximité de l'ouvre-bouche 7, les orifices 43 de passage d'air ont un effet favorisant l'écoulement lors de la distribution de produit dans le conduit de distribution 3. Les orifices 43 permettent de réaliser un effet venturi suite à l'injection de produit par l'orifice 39. L'air de la cavité buccale pénètre par les orifices 43 pour favoriser le transport efficace des particules de médicament au travers du conduit de distribution 3 et limiter ainsi la perte de médicament dans le conduit 3. Une telle configuration avec orifices 43 de passage d'air est particulièrement avantageuse dans un dispositif de distribution de produit 31' étanche à l'air, dans lequel l'organe de distribution 13' comporte une paroi interne 41, laquelle obture tout passage d'air extérieur en direction de l'ouverture de sortie 37, comme précédemment illustré sur la figure 6.

Un troisième mode de réalisation est illustré sur la figure 8, lequel est similaire au premier mode de réalisation précédemment décrit, à l'exception du fait que le conduit de distribution 3' se présente sous forme de cylindre creux à section circulaire. Ainsi, la paroi interne 8' du conduit de distribution 3' est conformée sous forme de cylindre droit à section transversale circulaire, de diamètre compris entre 20 et 30 millimètres, dans cet exemple égal à 20 millimètres.

Dans les modes de réalisation précédemment décrits, l'embout buccal 1 est composé d'au moins un matériau polymère. L'embout buccal 1 peut être fabriqué au moyen d'un procédé de moulage par injection.

Il est également décrit un procédé de distribution d'un produit au moyen d'un dispositif de distribution de produit 31, 31' précédemment décrit.

Un tel procédé de distribution comporte au moins les étapes suivantes :
- insertion de l'embout buccal 1 dans la bouche d'un utilisateur,
- insertion de la langue de l'utilisateur dans le logement lingual 5, et
- déclenchement de la distribution de produit lorsque la langue de l'utilisateur est en contact avec la surface de contact 15.

Il est à noter que cette dernière étape de déclenchement de la distribution peut être réalisée manuellement par l'utilisateur. Ainsi, l'utilisateur détectant que sa langue est en contact avec la surface de contact 15 peut déclencher la distribution de produit, par exemple par détection au moyen de la surface texturée 17.

Alternativement, lorsque la langue de l'utilisateur se trouve en contact avec la surface de contact 15, le dispositif de distribution de produit 31, 31' peut déclencher automatiquement la distribution de produit via les moyens de distribution 35 de produit de l'organe de distribution 13, 13'. La détection que la langue de l'utilisateur se trouve en contact avec la surface de contact 15 est réalisée par exemple par des moyens de détection de la position de la langue, comme par exemple un capteur de proximité ou un capteur de contact sur la surface de contact 15.

L'invention n'est pas limitée aux modes de réalisation présentés et d'autres modes de réalisation apparaîtront clairement à l'homme du métier. Il est notamment possible de combiner les deuxièmes et troisièmes modes de réalisation entre eux. L'exemple décrit dans le premier mode de réalisation est applicable par l'homme du métier aux deuxième et troisième modes de réalisation. Les dimensions indiquées sont celles correspondant à une utilisation par un utilisateur adulte. L'homme du métier saura adapter ces dimensions pour les adapter à une utilisation pédiatrique.

### Liste de références

1 : embout buccal
3, 3' : conduit de distribution
5 : logement lingual
7 : ouvre-bouche
8, 8' : paroi interne (du conduit de distribution 3, 3')
9 : ouverture de raccordement
11 : ouverture de distribution
13, 13' : organe de distribution
14 : ouverture linguale
15 : surface de contact
16 : surface latérale
17 : surface de fond
19 : partie supérieure (de la surface latérale 16)
21 : partie inférieure (de la surface périphérique 23)
23 : surface périphérique (du conduit de distribution 3)
25 : paroi périphérique (de l'ouvre-bouche 7)
27 : plaque
29 : surface d'appui frontale
31, 31' : dispositif de distribution de produit
33 : réservoir de produit
35 : moyens de distribution
37 : ouverture de sortie (de l'organe de distribution 13, 13')
38 : paroi périphérique
39 : buse de distribution
40 : ouverture d'entrée
41 : paroi interne
43 : orifice de passage d'air
45 : partie supérieure (de la surface périphérique 23)
P : plan de symétrie

## Revendications

1. Embout buccal (1) pour un dispositif de distribution de produit (31, 31'), comportant :
- un conduit de distribution (3, 3') de produit définissant un axe longitudinal (XX') et comprenant une ouverture de raccordement (9) configurée pour être raccordée à un organe de distribution (13, 13') du dispositif de distribution de produit (31, 31'), et une ouverture de distribution (11) configurée pour déboucher librement dans la bouche d'un utilisateur,
l'embout buccal (1) étant **caractérisé en ce qu'**il comporte :
- un logement lingual (5) configuré pour recevoir au moins une extrémité de la langue de l'utilisateur, comportant une ouverture linguale (14) autorisant l'insertion de la langue dans le logement lingual (5) et une surface de contact (15) configurée pour former une cible pour une extrémité de la langue de l'utilisateur.

2. Embout buccal (1) selon la revendication précédente, lequel comporte en outre un ouvre-bouche (7) entourant au moins en partie le conduit de distribution (3, 3') et le logement lingual (5).

3. Embout buccal (1) selon l'une quelconque des revendications précédentes, lequel comprend en outre une surface d'appui frontale (29) pour les lèvres de l'utilisateur.

4. Embout buccal (1) selon la revendication précédente, lequel comprend une plaque (27) comportant la surface d'appui frontale (29) et comportant une unique ouverture délimitant l'ouverture de raccordement (9).

5. Embout buccal (1) selon l'une quelconque des revendications 3 ou 4, dans lequel un angle formé entre l'axe longitudinal (XX') du conduit de distribution (3, 3') et la surface d'appui frontale (29) possède une valeur comprise entre 90 degrés et 120 degrés, de préférence entre 92 degrés et 100 degrés, plus préférentiellement voisine de 97,5 degrés.

6. Embout buccal (1) selon l'une quelconque des revendications précédentes, dans lequel le conduit de distribution (3, 3') comporte en outre au moins un orifice (43) de passage d'air, lequel est disposé préférentiellement sur la partie supérieure (45) du conduit de distribution (3, 3') opposée au logement lingual (5).

7. Embout buccal (1) selon l'une quelconque des revendications précédentes, dans lequel le logement lingual (5) comprend des moyens de détection de la position de la langue, de préférence un capteur de proximité ou un capteur de contact sur la surface de contact (15).

8. Embout buccal (1) selon l'une quelconque des revendications précédentes, dans lequel le logement lingual (5) comprend des moyens d'indication d'un positionnement correct de la langue de l'utilisateur, de préférence les moyens d'indication comprenant une surface texturée agencée sur la surface de contact (15).

9. Dispositif de distribution de produit (31, 31'), lequel comporte :
- un organe de distribution (13, 13'), et
- un embout buccal (1) selon l'une quelconque des revendications précédentes.

## Patentansprüche

1. Mundstück (1) für eine Produktabgabevorrichtung (31, 31'), aufweisend:
- einen Produktabgabekanal (3, 3'), der eine Längsachse (XX') definiert und eine Verbindungsöffnung (9), die konfiguriert ist, um mit einem Abgabeorgan (13, 13') der Produktabgabevorrichtung (31, 31') verbunden zu werden, und eine Abgabeöffnung (11) aufweist, die konfiguriert ist, um frei in den Mund eines Benutzers zu münden,
wobei das Mundstück (1) **dadurch gekennzeichnet ist, dass** es aufweist:
- eine Zungenaufnahme (5), die so konfiguriert ist, dass sie mindestens ein Ende der Zunge des Benutzers aufnimmt, mit einer lingualen Öffnung (14), die das Einführen der Zunge in die Zungenaufnahme (5) ermöglicht, und einer Kontaktfläche (15), die so konfiguriert ist, dass sie ein Ziel für ein Ende der Zunge des Benutzers bildet.

2. Mundstück (1) nach dem vorhergehenden Anspruch, welches zusätzlich einen Mundöffner (7) aufweist, der zumindest teilweise den Abgabekanal (3, 3') und die Zungenaufnahme (5) umgibt.

3. Mundstück (1) nach einem der vorhergehenden Ansprüche, das außerdem eine vordere Auflagefläche (29) für die Lippen des Benutzers aufweist.

4. Mundstück (1) nach dem vorhergehenden Anspruch, das eine Platte (27) aufweist, die die vordere Auflagefläche (29) aufweist und eine einzige Öffnung hat, die die Verbindungsöffnung (9) begrenzt.

5. Mundstück (1) nach einem der Ansprüche 3 oder 4, wobei ein Winkel zwischen der Längsachse (XX') des Abgabekanals (3, 3') und der vorderen Auflagefläche (29) einen Wert zwischen 90 Grad und 120 Grad, vorzugsweise zwischen 92 Grad und 100 Grad, besonders bevorzugt in der Nähe von 97,5 Grad, besitzt.

6. Mundstück (1) nach einem der vorhergehenden Ansprüche, wobei der Abgabekanal (3, 3') zusätzlich mindestens eine Luftdurchgangsöffnung (43) aufweist, die vorzugsweise an dem der Zungenaufnahme (5) gegenüberliegenden oberen Teil (45) des Abgabekanals (3, 3') angeordnet ist.

7. Mundstück (1) nach einem der vorhergehenden Ansprüche, wobei die Zungenaufnahme (5) Mittel zum Erfassen der Zungenposition aufweist, vorzugsweise einen Näherungssensor oder einen Kontaktsensor an der Kontaktfläche (15).

8. Mundstück (1) nach einem der vorhergehenden Ansprüche, wobei die Zungenaufnahme (5) Mittel zur Anzeige einer korrekten Positionierung der Zunge des Benutzers aufweist, wobei vorzugsweise die Anzeigemittel eine texturierte Oberfläche aufweisen, die auf der Kontaktfläche (15) angeordnet ist.

9. Produktabgabevorrichtung (31, 31'), aufweisend:
- ein Abgabeelement (13, 13'), und
- ein Mundstück (1) nach einem der vorhergehenden Ansprüche.

## Claims

1. Mouthpiece (1) for a product dispensing device (31, 31'), comprising :
- a product dispensing duct (3, 3') defining a longitudinal axis (XX') and comprising a connection opening (9) configured to be connected to a dispensing organ (13, 13') of the product dispensing device (31, 31'), and a dispensing opening (11) designed to open freely into the mouth of a user,
the mouthpiece (1) being **characterized in that** it comprises:
- a lingual cavity (5) configured to receive at least one end of the tongue of the user, comprising a lingual opening (14) allowing the tongue to be inserted into the lingual cavity (5) and a contact surface (15) configured to form a target for one end of the tongue of the user.

2. Mouthpiece (1) according to the preceding claim, further comprising a mouth opener (7) surrounding at least partially the dispensing duct (3, 3') and the lingual cavity (5).

3. Mouthpiece (1) according to any one of the preceding claims, further comprising a front support surface (29) for the lips of the user.

4. Mouthpiece (1) according to the preceding claim, comprising a plate (27) having the front support surface (29) and having a single opening delimiting the connection opening (9).

5. Mouthpiece (1) according to claim 3 or 4, wherein the value of an angle formed between the longitudinal axis (XX') of the dispensing duct (3, 3') and the front support surface (29) is between 90 degrees and 120 degrees, preferably between 92 degrees and 100 degrees, more preferably close to 97.5 degrees.

6. Mouthpiece (1) according to any one of the preceding claims, wherein the dispensing duct (3, 3') further comprises at least one air passage opening (43), which is preferably arranged on the upper part (45) of the dispensing duct (3, 3') opposite the lingual cavity (5).

7. Mouthpiece (1) according to any one of the preceding claims, wherein the lingual cavity (5) comprises means for detecting the position of the tongue, preferably a proximity sensor or a contact sensor on the contact surface (15).

8. Mouthpiece (1) according to any one of the preceding claims, wherein the lingual cavity (5) comprises indication means for indicating a correct positioning of the tongue of the user, preferably indication means comprising a textured surface arranged on the contact surface (15).

9. Product dispensing device (31, 31') comprising:
- a dispensing member (13, 13'), and
- a mouthpiece (1) according to any one of the preceding claims.
